# EUROPEAN PATENT APPLICATION

(11) **EP 2 065 474 A1**
(43) Date of publication of application: **03.06.2009**
(21) Application number: 07121810.1
(22) Date of filing: 28.11.2007
(51) Int. Cl.: C12Q 1/68

(54) **A method to assess prognosis and to predict therapeutic response to endocrine treatment**

(71) Applicant: Siemens Healthcare Diagnostics GmbH, 65760 Eschborn (DE)
(72) Inventor: Wirtz, Ralph, 50677 Köln (DE); Hennig, Guido, 50859 Köln (DE); von Törne, Christian, Dr., 42659 Solingen (DE)
(74) Representative: Maier, Daniel Oliver

(57) **Abstract**

The present invention is related to a method for predicting a clinical response of a patient suffering from or at risk of developing a neoplastic disease to a given mode of treatment by determination the status of at least one hormone receptor selected from the group comprising estrogen receptor, progesterone receptor and/or androgen receptor, said method comprising the steps of:
a) obtaining a biological sample from said patient;
b) determining the status of at least one hormone receptor selected from the group comprising estrogen receptor, progesterone receptor and/or androgen receptor in said sample, using at least two determinations selected from the group comprising:
aa) determining the expression level of the RNA transcripts of at least one gene encoding for a hormone receptor selected from the group comprising estrogen receptor, progesterone receptor and/or androgen receptor, and/or
bb) determining the DNA amplification level of at least one gene encoding for a hormone receptor selected from the group comprising estrogen receptor, progesterone receptor and/or androgen receptor, and/or
cc) determining the presence or absence of at least one single nucleotide polymorphism of at least one gene encoding for a hormone receptor selected from the group comprising estrogen receptor, progesterone receptor and/or androgen receptor;

c) comparing the pattern of expression level(s) of aa), amplification level(s) of bb) and/or the single nucleotide polymorphism(s) of cc) determined in step b) with one or several reference pattern(s) of expression and/or amplification levels and/or single nucleotide polymorphisms; and
d) predicting therapeutic outcome for said given mode of treatment in said patient from the outcome of the comparison in step c).

## Description

### Field of the invention

The present invention relates to methods to assess prognosis and to predict therapeutic response of a patient to cancer treatment.

### Background of the invention

Breast cancer is the most common type of cancer among women in Europe and in the United States, and belongs to the leading causes of cancer deaths among women.

It is known that estrogen promotes the growth of some cancers, such as breast cancer, especially those that express the estrogen receptor. In these cancers, the removal of estrogen or the blocking of its actions on the tumor cells can lead to tumor regression or prevent further tumor growth. Several therapies have been developed to block the effect of estrogen, or to lower estrogen levels in such patient, especially in estrogen receptor positive breast cancer patients, e.g. by administering of anti-estrogen drugs, such as tamoxifen.

Adjuvant and neoadjuvant endocrine therapy is the treatment of choice in hormone receptor-positive breast cancer. In addition, tamoxifen is first- and second-line endocrine therapy for hormone-sensitive advanced breast cancer.

Generally, drugs for endocrine therapy can be classified as either antagonists of estrogen binding to the estrogen receptor, or as inhibitors of estrogen biosynthesis, such as aromatase inhibitors. The most commonly used anti-estrogen drug is tamoxifen.

Other anti-estrogen drugs include raloxifene, which, like tamoxifen, blocks the effect of estrogen on breast tissue and breast cancer, toremifene, and megesterol, which is typically used for hormonal treatment of advanced breast cancer.

Anastrozole, an aromatase inhibitor, acts by preventing estrogen from activating its receptor, blocking an enzyme needed for production of estrogen. Anastrozole can be used in the adjuvant therapy setting and is also an option for women whose advanced breast cancer continues to grow during or after tamoxifen treatment.

However, the response of estrogen receptor positive patients to endocrine treatment varies, and no reliable predictors of responsiveness to endocrine treatment are available.

Currently, the probability of response of patients to a treatment with anti-estrogen drugs, such as tamoxifen, is usually determined by measuring the status of estrogen receptor on protein level. The current standard for the determination of estrogen receptor status is immunohistochemistry (IHC) by using subsequent diverse scoring systems, e. g. Allred or Remmele score.

Such assays based on protein-level measurements exhibit limited quantitative performance and comparatively high inter- and intra- assay variabilities. Especially immunohistochemistry often yields different results in different laboratories. Moreover, the final assessment is essentially subjective and is known to show substantial inter-operator, i.e. inter-pathologist, variance.

However, very limited amounts of estrogen receptor positive tumor cells (>1% of tumor cells) are known in the art to be sufficient to qualify the tumor to be estrogen receptor positive with the consequence that patients receive hormone therapy.

Still, a remarkable number of estrogen receptor positive patients as assessed by immunohistochemistry have no benefit after tamoxifen therapy and need additional or different treatment options if identified in advance.

However, response to endocrine therapy is comparatively low with only about 20% patients of hormone receptor positive breast cancer patients having benefit from endocrine treatment while a portion of the patients suffers severe side effects like adverse drug reactions (ADRs). In addition, there is a burden on national health systems due to the high cost of some therapies in this regime.

It is yet difficult to determine those patients which will respond to endocrine therapy. To date, there are no reliable response markers to predict response to endocrine therapy in breast cancer.

Therefore, a need exists to identify those patients who are likely to respond to endocrine therapy by providing predictive information. There is a great need for predictive methods that can assist the practicing physician to make treatment choices based on reliable analysis.

### Definitions

Unless defined otherwise, technical and scientific terms used herein have the same meaning as commonly understood by one of ordinary skill in the art to which this invention belongs.

The term "prediction" as used herein relates to the likelihood that a patient will respond either favorably or unfavorably to a given therapy. Especially, the term "prediction", as used herein, relates to an individual assessment of the malignancy of a tumor, or to the expected survival rate (DFS, disease free survival) of a patient, if the tumor is treated with a given therapy. In contrast thereto, the term "prognosis" relates to an individual assessment of the malignancy of a tumor, or to the expected survival rate (DFS, disease free survival) of a patient, if the tumor remains untreated.

The term "response marker" relates to a marker which can be used to predict the clinical response of a patient towards a given treatment. Response includes direct observation of tumor shrinkage upon neoadjuvant or palliative treatment as evident by e.g. CT-Scans and/or serum biomarkers as well as effects on Disease Free Survival (DFS), Overall Survival (OAS), Metastasis Specific Survival (MSS), Disease Specific Survival and related assessments.

The term "clinical response" of a patient, as used herein, relates to the effectiveness of a certain therapy in a patient, meaning an improvement in any measure of patient status, including those measures ordinarily used in the art, such as overall survival, progression free survival, recurrence-free survival, and distant recurrence-free survival. Recurrence-free survival (RFS) refers to the time from surgery to the first local, regional, or distant recurrence. Distant recurrence-free survival (DFRS) refers to the time from surgery to the first anatomically distant recurrence. The calculation of these measures in practice may vary from study to study depending on the definition of events to be either censored or not considered. The term "response marker" relates to a marker which can be used to predict the clinical response of a patient towards a given treatment.

The term "neoplastic disease" refers to a cancerous tissue this includes carcinomas, (e.g., carcinoma in situ, invasive carcinoma, metastatic carcinoma) and pre-malignant conditions, neomorphic changes independent of their histological origin (e.g. ductal, lobular, medullary, mixed origin).

The terms "cancer" and "cancerous" refer to or describe the physiological condition in mammals that is typically characterized by unregulated cell growth. The term "cancer" as used herein includes carcinomas, (e.g., carcinoma in situ, invasive carcinoma, metastatic carcinoma) and pre-malignant conditions, neomorphic changes independent of their histological origin. The term "cancer" is not limited to any stage, grade, histomorphological feature, invasiveness, aggressiveness or malignancy of an affected tissue or cell aggregation. In particular stage 0 cancer, stage I cancer, stage II cancer, stage III cancer, stage IV cancer, grade I cancer, grade II cancer, grade III cancer, malignant cancer, primary carcinomas, and all other types of cancers, malignancies and transformations associated with the lung, ovarian, cervix, esophagus, stomach, pancreas, prostate, head and neck, renal cell, colorectal or breast cancer are included. The terms "neoplastic disease" or "cancer" are not limited to any tissue or cell type. They also include primary, secondary or metastatic lesions of cancer patients, and also comprises lymph nodes affected by cancer cells or minimal residual disease cells either locally deposited or freely floating throughout the patient's body.

In addition to breast cancer examples of cancer include, but are not limited to, the group comprising gynecologic cancers selected from the group comprising ovarian cancer, vulvar cancer, vaginal cancer, tubal cancer, endometrian cancer and/or cervical cancer. As used herein, the term "gynecologic cancers" refers to cancer which are diagnosed in female reproductive organs that include the uterus, ovaries, cervix, fallopian tubes, vulva, and vagina. Examples of gynecologic cancers include, but are not limited to ovarian cancer, vulvar cancer, vaginal cancer, tubal cancer, endometrian cancer and/or cervical cancer. As used herein, the term "endometrian cancer", also called endometrial cancer or uterine cancer, includes malignant growth of cells in the endometrium, the lining of the uterus.

The term "tumor" as used herein, refers to all neoplastic cell growth and proliferation, whether malignant or benign, and all pre-cancerous and cancerous cells and tissues.

The term "neoplastic cells" refer to abnormal cells that grow by increased cellular proliferation, altered cell division symmetry or decreased cell death mechanisms more rapidly than normal. As such, neoplastic cells of the invention may be cells of a benign neoplasm or may be cells of a malignant neoplasm.

The term "endocrine treatment" refers to various treatment modalities known as hormonal therapy or anti-hormonal therapy that produce the desired therapeutic effect by means of change of hormone/hormones level. The treatment may include administration of hormones or hormone analogs, synthetic hormones or other drugs to the patient, or decreasing the level of hormones in the body by using hormone antagonists, or hormone ablation therapy either by surgical resection of ovaries or by chemical suppression of hormone synthesis. Endocrine therapy shall be taken to include hormonal therapies such as selective estrogen reuptake inhibitors, selective estrogen receptor downregulators, aromatase inhibitors and ovarian ablation. Preferred aromatase inhibitors are anastrozole, exemestane and letrozole. In addition fulvestrant can be used as an estrogen antagonist. In addition, agonists such as estrogen, progestin, gestagen may be used alone or in combination to treat cancer. In yet another addition, combinations of antagonists and agonists may be used alone or in combination to treat cancer.

The term "determining the status" as used herein, refers to a measurable property of a gene and its products, especially on the nucleotide level and the gene level including mutation status and gene expression status. A number of parameters to determine the status of a gene and its products can be used including, but not limited to, determining the level of protein expression, the amplification or expression status on RNA level or DNA level, of polynucleotides and of polypeptides, and the analysis of haplotype or the mutation status of the gene. An exemplary determinable property correlated with the status of estrogen receptor or progesterone receptor is the amount of the estrogen receptor or progesterone receptor RNA, DNA or other polypeptide in the sample or the presence of nucleotide polymorphisms.

The terms "biological sample" or "clinical sample", as used herein, refer to a sample obtained from a patient. The sample may be of any biological tissue or fluid. Such samples include, but are not limited to, sputum, blood, serum, plasma, blood cells (e.g., white cells), tissue, core or fine needle biopsy samples, cell-containing body fluids, free floating nucleic acids, urine, peritoneal fluid, and pleural fluid, liquor cerebrospinalis, tear fluid, or cells there from. Biological samples may also include sections of tissues such as frozen or fixed sections taken for histological purposes or microdissected cells or extracellular parts thereof. A biological sample to be analyzed is tissue material from a neoplastic lesion taken by aspiration or punctuation, excision or by any other surgical method leading to biopsy or resected cellular material. Such a biological sample may comprise cells obtained from a patient. The cells may be found in a cell "smear" collected, for example, by a nipple aspiration, ductal lavarge, fine needle biopsy or from provoked or spontaneous nipple discharge. In another embodiment, the sample is a body fluid. Such fluids include, for example, blood fluids, cerebrospinal fluid, serum, plasma, lymph, ascitic fluids, gynecologicalal fluids, or urine but not limited to these fluids.

By "array" is meant an arrangement of addressable locations or "addresses" on a device. The locations can be arranged in two dimensional arrays, three dimensional arrays, or other matrix formats. The number of locations can range from several to at least hundreds of thousands. Most importantly, each location represents an independent reaction site. Arrays include but are not limited to nucleic acid arrays, protein arrays and antibody arrays. A "nucleic acid array" refers to an array containing nucleic acid probes, such as oligonucleotides, polynucleotides or larger portions of genes. The nucleic acid on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about 100/cm², and preferably at least about 1000/cm². The regions in a microarray have typical dimensions, e.g., diameters, in the range of between about 10-250 •m, and are separated from other regions in the array by about the same distance.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides are preferably single-stranded DNA probe oligonucleotides.

The terms "modulated" or "modulation" or "regulated" or "regulation" and "differentially regulated" as used herein refer to both upregulation, i.e., activation or stimulation, e.g., by agonizing or potentiating, and down regulation, i.e., inhibition or suppression, e.g., by antagonizing, decreasing or inhibiting.

The term "expression levels" refers, e.g., to a determined level of gene expression. The term "pattern of expression levels" refers to a determined level of gene expression compared either to a reference gene, e.g. housekeeper or inversely regulated genes, or to a computed average expression value, e.g. in DNA-chip analyses. A pattern is not limited to the comparison of two genes but is more related to multiple comparisons of genes to reference genes or samples. A certain "pattern of expression levels" may also result and be determined by comparison and measurement of several genes disclosed hereafter and display the relative abundance of these transcripts to each other.

Alternatively, a differentially expressed gene disclosed herein may be used in methods for identifying reagents and compounds and uses of these reagents and compounds for the treatment of cancer as well as methods of treatment. The differential regulation of the gene is not limited to a specific cancer cell type or clone, but rather displays the interplay of cancer cells, muscle cells, stromal cells, connective tissue cells, other epithelial cells, fat cells, endothelial cells of blood vessels as well as cells of the immune system, e.g. lymphocytes, macrophages, killer cells.

The term "RNA expression level" refers to a determined level of the converted DNA gene sequence information into transcribed RNA, the initial unspliced RNA transcript or the mature mRNA. RNA expression can be monitored by measuring the levels of either the entire RNA of the gene or subsequences.

The term "DNA amplification level" refers to a determined level of the process by which multiple copies of a gene or gene fragment are formed in a particular cell or cell line.

The term "pattern of RNA expression" refers to a determined level of RNA expression compared either to a reference RNA or to a computed average expression value. The term "pattern of DNA amplification" refers to a determined level of DNA amplification compared either to a reference DNA or to a computed average expression value. A pattern is not limited to the comparison of two RNAs or DNAs but is more related to multiple comparisons of RNAs or DNAs to reference RNAs or DNAs or samples. A certain "pattern of expression levels" or "pattern of DNA amplification" may also result and be determined by comparison and measurement of several RNAs or DNAs and display the relative abundance of these transcripts to each other.

A "reference pattern of expression levels", within the meaning of the invention shall be understood as being any pattern of expression levels that can be used for the comparison to another pattern of expression levels. In a preferred embodiment of the invention, a reference pattern of expression levels is, e.g., an average pattern of expression levels observed in a group of healthy or diseased individuals, serving as a reference group. Accordingly, a "reference pattern of amplification levels", within the meaning of the invention shall be understood as being any pattern of amplification levels that can be used for the comparison to another pattern of amplification levels. In a preferred embodiment of the invention, a reference pattern of amplification levels is, e.g., an average pattern of amplification levels observed in a group of healthy or diseased individuals, serving as a reference group.

"Primer pairs" and "probes", within the meaning of the invention, shall have the ordinary meaning of this term which is well known to the person skilled in the art of molecular biology. In a preferred embodiment of the invention "primer pairs" and "probes", shall be understood as being polynucleotide molecules having a sequence identical, complementary, homologous, or homologous to the complement of regions of a target polynucleotide which is to be detected or quantified. In yet another embodiment nucleotide analogues are also comprised for usage as primers and/or probes. Probe technologies used for kinetic or real time PCR applications could be e.g. TaqMan^{®} systems obtainable at Roche Molecular Diagnostics, extension probes such as Scorpion^{®} Primers, Dual Hybridisation Probes, Amplifluor^{®} obtainable at Chemicon International, Inc, or Minor Groove Binders.

"Individually labeled probes", within the meaning of the invention, shall be understood as being molecular probes comprising a polynucleotide, oligonucleotide or nucleotide analogue and a label, helpful in the detection or quantification of the probe. Preferred labels are fluorescent molecules, luminescent molecules, radioactive molecules, enzymatic molecules and/or quenching molecules.

"Arrayed probes", within the meaning of the invention, shall be understood as being a collection of immobilized probes, preferably in an orderly arrangement. In a preferred embodiment of the invention, the individual "arrayed probes" can be identified by their respective position on the solid support, e.g., on a "chip".

The phrase "response", "therapeutic outcome", or "response to therapy" refers, in the given therapeutic setting including endocrine regimens to the observation of a defined tumor free or recurrence free survival time (e.g. 2 years, 4 years, 5 years, 10 years). This time period of disease free survival may vary among the different tumor entities but is sufficiently longer than the average time period in which most of the recurrences appear. In a neo-adjuvant and palliative therapy modality, response may additionally be monitored by measurement of tumor shrinkage and regression due to apoptosis and necrosis of the tumor mass or reduced blood supply due to altered angiogenic events.

The term "recurrence" or " recurrent disease" includes distant metastasis that can appear even many years after the initial diagnosis and therapy of a tumor, or local events such as infiltration of tumor cells into regional lymph nodes, or occurrence of tumor cells at the same site and organ of origin within an appropriate time.

"Prediction of recurrence" or "prediction of therapeutic outcome" does refer to the methods described in this invention, wherein a tumor specimen is analyzed for e.g. its gene expression, genomic status and/or histopathological parameters, such as the TNM Classification of Malignant Tumours (TNM), a cancer staging system developed and maintained by the International Union Against Cancer and Grade, and/or imaging data and furthermore classified based on correlation of the expression pattern to known ones from reference samples. This classification may either result in the statement that such given tumor will develop recurrence and therefore is considered as a "non responding" tumor to the given therapy, or may result in a classification as a tumor with a prolonged disease free post therapy time.

"Biological activity" or "bioactivity" or "activity" or "biological function", which are used interchangeably, herein mean an effector or antigenic function that is directly or indirectly exerted by a polypeptide (whether in its native or denatured conformation), or by any fragment thereof *in vivo* or *in vitro.* Biological activities include but are not limited to binding to polypeptides, binding to other proteins or molecules, enzymatic activity, signal transduction, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "marker" or "biomarker" refers to a biological molecule, e.g., a nucleic acid, peptide, protein, hormone, etc., whose presence or concentration can be detected and correlated with a known condition, such as a disease state or a combination of these, e.g. by a mathematical algorithm.

The term "marker gene" as used herein, refers to a differentially expressed gene whose expression pattern may be utilized as part of a predictive, prognostic or diagnostic process in malignant neoplasia or cancer evaluation, or which, alternatively, may be used in methods for identifying compounds useful for the treatment or prevention of malignant neoplasia and head and neck, colon or breast cancer in particular. A marker gene may also have the characteristics of a target gene.

"Target gene", as used herein, refers to a differentially expressed gene involved in cancer, e.g. breast cancer in a manner in which modulation of the level of the target gene expression or of the target gene product activity may act to ameliorate symptoms of malignant neoplasia. A target gene may also have the characteristics of a marker gene.

The term "receptor", as used herein, relates to a protein on the cell membrane or within the cytoplasm or cell nucleus that binds to a specific molecule, especially a hormone as estrogen or progesterone, and initiates the cellular response.

The term "signalling pathway" is related to any intra- or intercellular process by which cells converts one kind of signal or stimulus into another, most often involving ordered sequences of biochemical reactions out- and inside the cell, that are carried out by enzymes and linked through hormones and growth factors (intercellular), as well as second messengers (intracellular), the latter resulting in what is thought of as a "second messenger pathway". In many signalling pathways, the number of proteins and other molecules participating in these events increases as the process emanates from the initial stimulus, resulting in a "signal cascade" and often results in a relatively small stimulus eliciting a large response.

The term "small molecule", as used herein, is meant to refer to a compound which has a molecular weight of less than about 5 kD and most preferably less than about 4 kD. Small molecules can be nucleic acids, peptides, polypeptides, peptidomimetics, carbohydrates, lipids or other organic (carbon- containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

When used in reference to a single-stranded nucleic acid sequence, the term "substantially homologous" refers to any probe that can hybridize (i.e., it is the complement of) the single-stranded nucleic acid sequence under conditions of low stringency as described above.

As used herein, the term "hybridization" is used in reference to the pairing of complementary nucleic acids.

The term "hybridization based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. These approaches are also known as "array based methods". Yet another hybridization based method is PCR, which is described below. When it comes to the determination of expression levels, hybridization based methods may for example be used to determine the amount of mRNA for a given gene.

The term "a PCR based method" as used herein refers to methods comprising a polymerase chain reaction (PCR). This is an approach for exponentially amplifying nucleic acids, like DNA or RNA, via enzymatic replication, without using a living organism. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR). The term "PCR based method" comprises both end-point PCR applications as well as kinetic/real time PCR techniques applying special fluorophors or intercalating dyes which emit fluorescent signals as a function of amplified target and allow monitoring and quantification of the target. Quantification methods could be either absolute by external standard curves or relative to a comparative internal standard.

The term "method based on the electrochemical detection of molecules" relates to methods which make use of an electrode system to which molecules, particularly biomolecules like proteins, nucleic acids, antigens, antibodies and the like, bind under creation of a detectable signal. Such methods are for example disclosed in WO0242759, WO0241992 and WO02097413 filed by the applicant of the present invention, the content of which is incorporated by reference herein. These detectors comprise a substrate with a planar surface which is formed, for example, by the crystallographic surface of a silicon chip, and electrical detectors which may adopt, for example, the shape of interdigital electrodes or a two dimensional electrode array. These electrodes carry probe molecules, e.g. nucleic acid probes, capable of binding specifically to target molecules, e.g. target nucleic acid molecules. The probe molecules are for example immobilized by a Thiol-Gold-binding. For this purpose, the probe is modified at its 5'- or 3' -end with a thiol group which binds to the electrode comprising a gold surface. These target nucleic acid molecules may carry, for example, an enzyme label, like horseradish peroxidise (HRP) or alkaline phosphatase. After the target molecules have bound to the probes, a substrate is then added (e.g., •-naphthyl phosphate or 3,3'5,5'-tetramethylbenzidine which is converted by said enzyme, particularly in a redox-reaction. The product of said reaction, or a current generated in said reaction due to an exchange of electrons, can then be detected with help of the electrical detector in a site specific manner.

The term "anamnesis" relates to patient data gained by a physician or other healthcare professional by asking specific questions, either of the patient or of other people who know the person and can give suitable information (in this case, it is sometimes called heteroanamnesis), with the aim of obtaining information useful in formulating a diagnosis and providing medical care to the patient. This kind of information is called the symptoms, in contrast with clinical signs, which are ascertained by direct examination.

The term "etiopathology" relates to the course of a disease, that is its duration, its clinical symptoms, and its outcome.

The term "nucleic acid molecule" is intended to indicate any single- or double stranded nucleic acid and/or analogous molecules comprising DNA, cDNA and/or genomic DNA, RNApreferably mRNA, peptide nucleic acid (PNA), locked nucleic acid (LNA) and/or Morpholino.

The term "stringent conditions" relates to conditions under which a probe will hybridize to its target subsequence, but to no other sequences. Stringent conditions are sequence-dependent and will be different in different circumstances. Longer sequences hybridize specifically at higher temperatures. Generally, stringent conditions are selected to be about 5° C. lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. (As the target sequences are generally present in excess, at Tm, 50% of the probes are occupied at equilibrium). Typically, stringent conditions will be those in which the salt concentration is less than about 1.0 M Na ion, typically about 0.01 to 1.0 M Na ion (or other salts) at pH 7.0 to 8.3 and the temperature is at least about 30° C. for short probes (e.g. 10 to 50 nucleotides) and at least about 60° C. for longer probes. Stringent conditions may also be achieved with the addition of destabilizing agents, such as formamide and the like.

The term "fragment of the nucleic acid molecule" is intended to indicate a nucleic acid comprising a subset of a nucleic acid molecule according to one of the claimed sequences. The same is applicable to the term "fraction of the nucleic acid molecule".

The term "variant of the nucleic acid molecule" refers herein to a nucleic acid molecule which is substantially similar in structure and biological activity to a nucleic acid molecule according to one of the claimed sequences.

The term "homologue of the nucleic acid molecule" refers to a nucleic acid molecule the sequence of which has one or more nucleotides added, deleted, substituted or otherwise chemically modified in comparison to a nucleic acid molecule according to one of the claimed sequences, provided always that the homologue retains substantially the same binding properties as the latter.

The term "derivative" as used herein, refers to a nucleic acid molecule that has similar binding characteristics to a target nucleic acid sequence as a nucleic acid molecule according to one of the claimed sequences

The term "hybridizing counterparts" as used herein, refers to a nucleic acid molecule that is capable of hybridizing to a nucleic acid molecules under stringent conditions.

The term "single nucleotide polymorphism" (SNP) refers to a DNA sequence having a polymorphic site occupied by a single nucleotide, which is the site of variation between allelic sequences. Typically the polymorphic site is occupied by a base other than the reference base. Single nucleotide polymorphisms may occur in protein-coding nucleic acid sequences, in which case such variations in the DNA sequence may give rise to a defective or otherwise variant protein, or effect the development of genetic disease as cancer.

As used herein, the term "allele" refers to a variant form of a given sequence (e.g., including but not limited to, genes containing one or more SNPs). A large number of genes are present in multiple allelic forms in a population. A diploid organism carrying two different alleles of a gene is said to be heterozygous for that gene, whereas a homozygote carries two copies of the same allele.

### Object of the invention

It is one object of the present invention to provide an improved method for predicting outcome of cancer patients by providing prognostic and or predictive information for endocrine treatment concerning the therapeutic outcome of a given treatment in breast cancer and gynaecological cancers including surgery, systemic and/or local application of chemotherapeutic and/or endocrine agents as well as antibody based, nucleic acid based and/or small molecule based strategies.

It is another object of the present invention to provide a method for predicting a clinical response of breast cancer and gynaecological cancers to a given treatment based on tissue analysis before, during or after therapy.

These objects are met with the methods and means according to the independent claims of the present invention. The dependent claims are related to preferred embodiments.

### Summary of the invention

Before the invention is described in detail, it is to be understood that this invention is not limited to the particular component parts of the process steps of the methods described as such methods may vary. It is also to be understood that the terminology used herein is for purposes of describing particular embodiments only, and is not intended to be limiting. It must be noted that, as used in the specification and the appended claims, the singular forms "a" "an" and "the" include singular and/or plural referents unless the context clearly dictates otherwise. It is also to be understood that plural forms include singular and/or plural referents unless the context clearly dictates otherwise. It is moreover to be understood that, in case parameter ranges are given which are delimited by numeric values, the ranges are deemed to include these limitation values.

According to the invention, a method is provided for predicting a clinical response of a patient suffering from or at risk of developing a neoplastic disease to a given mode of treatment by determination the status of at least one hormone receptor selected from the group comprising estrogen receptor, progesterone receptor and/or androgen receptor, said method comprising the steps of:
a) obtaining a biological sample from said patient;
b) determining the status of at least one hormone receptor selected from the group comprising estrogen receptor, progesterone receptor and/or androgen receptor in said sample, using at least two determinations selected from the group comprising:
   aa) determining the expression level of the RNA transcripts of at least one gene encoding for a hormone receptor selected from the group comprising estrogen receptor, progesterone receptor and/or androgen receptor, and/or
   bb) determining the DNA amplification level of at least one gene encoding for a hormone receptor selected from the group comprising estrogen receptor, progesterone receptor and/or androgen receptor, and/or
   cc) determining the presence or absence of at least one single nucleotide polymorphism of at least one gene encoding for a hormone receptor selected from the group comprising estrogen receptor, progesterone receptor and/or androgen receptor;
c) comparing the pattern of expression level(s) of aa), amplification level(s) of bb) and/or the single nucleotide polymorphism(s) of cc) determined in step b) with one or several reference pattern(s) of expression and/or amplification levels and/or single nucleotide polymorphisms; and
d) predicting therapeutic outcome for said given mode of treatment in said patient from the outcome of the comparison in step c).

The method may be used to determine whether a patient especially a woman is likely to respond to a given mode of treatment especially endocrine treatment. This means that, for example, the invention provides the possibility to specifically determine with better sensitivity a neoplastic disease in a patient characterized by an elevated expression level of a hormone receptor selected from the group comprising estrogen receptor, progesterone receptor and/or androgen receptor and/or single nucleotide polymorphism. On the basis of this finding it can be selected if endocrine treatment is the most promising therapy for the respective patient.

Furthermore the method according to the invention may be applied in both adjuvant, neoadjuvant and metastatic settings.

In a preferred embodiment the given mode of treatment is endocrine treatment.

Said given mode of treatment can be selected from the group comprising endocrine treatment, chemotherapy, administration of small molecule inhibitors, antibody based regimen, anti-proliferation regimen, pro-apoptotic regimen, pro-differentiation regimen, radiation and/or surgical therapy or a combination of these.

Said chemotherapy may comprise the administration of at least one agent selected from the group comprising Cyclophosphamid (Endoxan®, Cyclostin®). Adriamycin (Doxorubicin) (Adriblastin®), BCNU (Carmustin) (Carmubris®), Busulfan (Myleran®),Bleomycin (Bleomycin®), Carboplatin (Carboplat®), Chlorambucil (Leukeran®), Cis-Platin (Cisplatin®), Platinex (Platiblastin®), Dacarbazin (DTIC®;Detimedac®), Docetaxel (Taxotere®), Epirubicin (Farmorubicin®), Etoposid (Vepesid®), 5-Fluorouracil (Fluroblastin®, Fluorouracil®), Gemcitabin (Gemzar®), Ifosfamid (Holoxan®), Interferon alpha (Roferon®), Irinotecan (CPT 11, Campto®), Melphalan (Alkeran®),Methotrexat (Methotrexat®, Farmitrexat®), Mitomycin C (Mitomycin®), Mitoxantron (Novantron®), Oxaliplatin (Eloxatine®), Paclitaxel (Taxol®), Prednimustin (Sterecyt®), Procarbazin (Natulan®), Pemetrexed (Alimta®), Ralitrexed (Tomudex®), Topotecan (Hycantin®), Trofosfamid (Ixoten®), Vinblastin (Velbe®), Vincristin (Vincristin®), Vindesin (Eldisine®) and/or Vinorelbin (Navelbine®).

In a preferred embodiment of the invention, said given mode of chemotherapy is targeted therapy such as small molecule inhibitors like Sunitinib (tradename Sutent®), Sorafenib (tradename Nexavar®), Lapatinib (Tykerb®) and/or therapeutic antibodies, e.g. Bevacizumab (tradename Avastin®) or cetuximab (tradename Erbitux®).

However, other treatments related to signalling pathways which fall under the scope of the present invention comprise the administration of Sorafenib (tradename Nexavar®, BAY 43-9005, target receptors are VEGFR-2, VEGFR-3, c-KIT, PDGFR-B, RET and Raf-Kinase), BAY 57-9352 (target receptor is VEGFR-2), Sunitinib (tradename Sutent®, target receptors are VEGFR-1, VEGFR-2 and PDGFR), AG13925 (target receptors are VEGFR-1 and VEGFR-2), AG013736 (target receptors are VEGFR-1 and VEGFR-2), AZD2171 (target receptors are VEGFR-1 and VEGFR-2), ZD6474 (target receptors are VEGFR-1, VEGFR-2 and VEGFR-3), PTK-787/ZK-222584 (target receptors are VEGFR-1 and VEGFR-2), CEP-7055 (target receptors are VEGFR-1, VEGFR-2 and VEGFR-3), CP-547 (target receptors are VEGFR-1 and VEGFR-2), CP-632 (target receptors are VEGFR-1 and VEGFR-2), GW786024 (target receptors are VEGFR-1, VEGFR-2 and VEGFR-3), AMG706 (target receptors are VEGFR-1, VEGFR-2 and VEGFR-3), Imatinib mesylate (tradename Glivec®/Gleevec®, target receptors are berabl and c-KIT), BMS-214662 (target enzyme is Ras farnesyl transferase), CCI-779 (target enzyme is mTOR), RAD0001 (tradename everolismus®, target enzyme is mTOR), CI-1040 (target enzyme is MEK), SU6668 (target receptors are VEGFR-2, PDGFR-B and FGFR-1)" AZD6126, CP547632 (target receptors are VEGFRs), CP868596 GW786034 (target receptors are PDGFRs), ABT-869 (target receptors are VEGFRs and PDGFRs), AEE788 (target receptors are VEGFRs and PDGFRs), AZD0530 (target enzymes are src and abl), and CEP7055.

In a preferred embodiment the at least two determinations include determining the expression level of the RNA transcripts of at least one gene encoding for a hormone receptor selected from the group comprising estrogen receptor, progesterone receptor and/or androgen receptor, and determining the DNA amplification level of at least one gene encoding for a hormone receptor selected from the group comprising estrogen receptor, progesterone receptor and/or androgen receptor.

In another preferred embodiment the at least two determinations include determining the expression level of the RNA transcripts of at least one gene encoding for a hormone receptor selected from the group comprising estrogen receptor, progesterone receptor and/or androgen receptor, and determining the presence or absence of at least one single nucleotide polymorphism of at least one gene encoding for a hormone receptor selected from the group comprising estrogen receptor, progesterone receptor and/or androgen receptor.

In yet another preferred embodiment the at least two determinations include determining the DNA amplification level of at least one gene encoding for a hormone receptor selected from the group comprising estrogen receptor, progesterone receptor and/or androgen receptor, and determining the presence or absence of at least one single nucleotide polymorphism of at least one gene encoding for a hormone receptor selected from the group comprising estrogen receptor, progesterone receptor and/or androgen receptor.

A combinatorial analysis of hormone receptor status selected from the group comprising estrogen receptor, progesterone receptor and/or androgen receptor status on two different levels, especially on RNA and DNA level has the advantage to significantly improve sensitivity and specificity of predicting a clinical response.

In a even more preferred embodiment, a combinatorial analysis of hormone receptor status selected from the group comprising estrogen receptor, progesterone receptor and/or androgen receptor status on three different levels, on RNA and DNA level and by determining single nucleotide polymorphism is provided.

A combinatorial analysis of hormone receptor status selected from the group comprising estrogen receptor, progesterone receptor and/or androgen receptor status on three different levels, on RNA and DNA level and by determining single nucleotide polymorphism will further enhance sensitivity and/or specificity and/or negative predictive value and/or positive predictive value of predicting a clinical response.

An analysis of hormone receptor status selected from the group comprising estrogen receptor, progesterone receptor and/or androgen receptor status on three different levels will provide a highly enhanced sensitivity and/or specificity and/or negative predictive value and/or positive predictive value of predicting a clinical response.

The inventors suggest, for the first time, to use a combinatorial analysis of hormone receptor status selected from the group comprising estrogen receptor, progesterone receptor and/or androgen receptor status on at least two different levels, or on three different levels for the prediction of therapeutic outcome of a given mode of treatment, especially endocrine treatment.

The improved sensitivity and/or specificity and/or negative predictive value and/or positive predictive value allows for example to identity a breast cancer population which has superior response to endocrine therapies and may therefore be spared from chemotherapy or other costly or harsh regimens.

In this regard, a better detection especially of estrogen receptor status enables to identify ESR1 positive tumors that cannot be resolved by immunohistochemical techniques. The method according to the invention can help to detect tumors which are most susceptible to a given mode of treatment especially to endocrine treatment, but which have so far remained undetected with methods from the state of the art.

In a especially preferred embodiment the at least two determinations include determining the expression level of the RNA transcripts of a gene encoding for the estrogen receptor and determining the DNA amplification level of a gene encoding for estrogen receptor.

Especially the combination of the additional information based on the highly variable RNA expression of ESR1 as assessed by RT-PCR can improve the prediction of endocrine treatment responsive tumors compared to DNA analysis alone.

Therefore, the method could substitute currently available measurements, or used in addition to currently available tests to make diagnosis more accurate.

In a preferred embodiment of this invention the expression of ESR1 can be utilized for discrimination of responders and non-responders to a given treatment, especially endocrine treatment.

In preferred embodiments the method is a method for predicting a favorable response to endocrine treatment.

With "estrogen receptor" all different forms of the receptor are included, preferably two different forms of the estrogen receptor, usually referred to as alpha and beta, each encoded by a separate gene (ESR1 and ESR2 respectively).

In a preferred embodiment the genes encoding for estrogen receptor are selected from the group comprising ESR1 and/or ESR2.

In a preferred embodiment of the present invention, it is provided that the gene encoding for the estrogen receptor which is determined is ESR1 (estrogen receptor 1). Estrogen receptor 1 has the advantage that it represents an essential marker of breast cancer.

In a preferred embodiment of the present invention, it is provided that the gene encoding for the estrogen receptor which is determined is ESR2 (estrogen receptor 2). Estrogen receptor 2 has the advantage that it represents an essential marker of breast cancer.

In a preferred embodiment of the present invention, it is provided that the gene encoding for the progesterone receptor which is determined is PGR (progesterone receptor). Progesterone receptor has the advantage that it represents an essential marker of breast cancer.

In a preferred embodiment of the present invention, it is provided that the gene encoding for the androgen receptor which is determined is AR (androgen receptor). Androgen receptor has the advantage that it represents an essential marker of breast cancer.

In another preferred embodiment of the present invention, it is provided that the single nucleotide polymorphisms of estrogen receptor are selected from the group of dbSNP Submission_ID comprising rs2071454, rs28462265, rs3138774, rs9371556, rs9340770, rs9340771, rs867239, rs867240, rs34418993, rs41291045, rs9340772, rs872921, rs2077647, rs9340773, rs746432, rs17847065, rs17847076, rs9340802, rs4986934, rs1801132, rs17847067, rs9341068, rs9341069, rs34840398, rs2228480, rs9340799, and/or rs2234693, as shown in Table 1.

In yet another preferred embodiment of the present invention, it is provided that the single nucleotide polymorphisms of progesterone receptor are selected from the group of dbSNP Submission_ID comprising rs34720665, rs11571142, rs11571141, rs 10895068, rs2008112, rs518162, rs11571140, rs11571139, rs11224606, rs11571138, rs625942, rs11571137, rs35974050, rs11571136, rs500760, rs11571252, rs2020880, rs2020878, rs1042839, rs1042838, rs11571222, rs2020874, rs2020876, rs11571179, rs11571152, rs11571151, rs11571150, rs1379130, rs11571149, rs11571148, rs11571147, rs3740753, rs35747049, rs34852313, rs11571146, rs10160588, rs10160726, rs36055552, rs11571145, rs11571144, rs3740754, and/or rs11571143, as shown in Table 2.

In another preferred embodiment of the present invention, it is provided that upregulated expression of the RNA transcripts of at least one gene encoding for a hormone receptor selected from the group comprising estrogen receptor, progesterone receptor and/or androgen receptor, and/or of the DNA amplification level of a hormone receptor selected from the group comprising estrogen receptor, progesterone receptor and/or androgen receptor, and/or determining of the presence or absence of at least one single nucleotide polymorphism of a hormone receptor selected from the group comprising estrogen receptor, progesterone receptor and/or androgen receptor determined in step b) is indicative of a prediction as regards therapeutic good outcome.

In yet another preferred embodiment of the present invention, the aforementioned treatment is an endocrine treatment, such as (but not limited to) tamoxifen or aromatase inhibitors. It is of note that the predicition of benefit to one therapeutical does not necessarily imply beneficial prediction for any other therapeuticals, e.g. a marker for benfit from tamoxifen treatment does not necessarily imply benefit from aromatase treatment in the same patient.

In yet another preferred embodiment of the present invention, RNA over-expression and/or DNA amplification is indicative for treatment benefit.

In a preferred embodiment of the present invention, the presence of at least one SNP is indicative for treatment benefit, e.g. homozygous or heterozygous.

In a preferred embodiment of the present invention, the absesence of at least one SNP is indicative for treatment benefit, e.g. wild-type homozygous.

In another preferred embodiment, at least one absent and at least one present SNP is indicative for treatment benefit.

Preferably, upregulated expression of the RNA transcripts of ESR1 and upregulated expression of the DNA amplification level of ESR1 is indicative of a promising prediction as regards therapeutic outcome for endocrine treatment.

More preferably, upregulated expression of the RNA transcripts of ESR1 and upregulated expression of the DNA amplification level of ESR1 and the absence or presence of at least one single nucleotide polymorphism of estrogen receptor selected from the group comprising rs746432, rs1801132, rs9340799, rs2234693, rs9340773, rs17847065 and/or rs17847076 is indicative of a promising prediction as regards therapeutic outcome for endocrine treatment.

Other parameters may as well be used and combined in order to predict the therapeutic outcome for endocrine treatment. In another preferred embodiment of the present invention, it is provided that in step b) additionally the presence and/or absence of at least one single nucleotide polymorphism of at least one tamoxifen metabolism gene is determined.

It is advantageous that the additional information on drug metabolism can further improve the sensitivity and/or specificity and/or positive predictive value and/or negative predictive value of the method according to the present invention.

It is provided in an another preferred embodiment of the present invention that said tamoxifen metabolism genes are selected from the group comprising CYP2D6, SULT1A1, UGT2B15, CYP2C19, CYP3A5, CYP2B6, and/or CYP2C9.

It can be especially advantageous that by a combined determination of the expression level of the RNA transcripts of at least one gene encoding for a hormone receptor selected from the group comprising estrogen receptor, progesterone receptor and/or androgen receptor, the DNA amplification level of at least one gene encoding for a hormone receptor selected from the group comprising estrogen receptor, progesterone receptor and/or androgen receptor, and/or the presence or absence of at least one single nucleotide polymorphism of at least one gene encoding for a hormone receptor selected from the group comprising estrogen receptor, progesterone receptor and/or androgen receptor, together with a determination of the presence or absence of at least one single nucleotide polymorphism of at least one tamoxifen metabolism gene a subpopulation of patients that is ESR1 positive can be classified as a responder or non-responder under endocrine treatment. This information may be useful for the selection of treatment options.

When additionally the presence of at least one single nucleotide polymorphism of at least one tamoxifen metabolism gene selected from the group comprising CYP2D6, SULT1A1, UGT2B15, CYP2C19, CYP3A5, CYP2B6, and/or CYP2C9 is determined, it is advantageously possible to determine a subpopulation of patients that is ESR1 positive and will respond to tamoxifen treatment.

Moreover, it is advantageously possible, to determine a subpopulation of patients that does not have benefit from either endocrine therapy and should receive an alternative therapy, such as chemotherapy, a different endocrine therapy, targeted therapy, or other treatment options.

In yet another preferred embodiment of the present invention, it is provided that in step b) the status of estrogen receptor is determined comprising the steps of:
aa) determining the RNA expression level of estrogen receptor, and
bb) determining the DNA amplification level of estrogen receptor, and
cc) determining the presence or absence of at least one single nucleotide polymorphism of estrogen receptor.

Preferably, the ESR1 expression and amplification status is determined. More preferably, additionally, the the presence or absence of at least one single nucleotide polymorphism of ESR1 is determined.

In especially preferred embodiment of the present invention, it is provided that in step b) the status of ESR1 is determined comprising the steps of:
aa) determining the RNA expression level of ESR1, and
bb) determining the DNA amplification level of ESR1, and
cc) determining the presence or absence of at least one single nucleotide polymorphism of ESR1.

In especially preferred embodiment of the present invention, it is provided that in step b) the status of ESR2 is determined comprising the steps of:
aa) determining the RNA expression level of ESR2, and
bb) determining the DNA amplification level of ESR2, and
cc) determining the presence or absence of at least one single nucleotide polymorphism of ESR2.

In especially preferred embodiment of the present invention, it is provided that in step b) the status of PGR is determined comprising the steps of:
aa) determining the RNA expression level of PGR, and
bb) determining the DNA amplification level of PGR, and
cc) determining the presence or absence of at least one single nucleotide polymorphism of PGR.

In especially preferred embodiment of the present invention, it is provided that in step b) the status of AR is determined comprising the steps of:
aa) determining the RNA expression level of AR, and
bb) determining the DNA amplification level of AR, and
cc) determining the presence or absence of at least one single nucleotide polymorphism of AR.

It is particularly preferred that, in the method according to the invention, said expression level and/or amplification level and/or presence and/or absence of at least one single nucleotide polymorphism determined in step b) are determined by
a) a hybridization based method;
b) a PCR based method;
c) a method based on the electrochemical detection of particular molecules, and/or by
d) an array-based method.

The above mentioned methods have in common that they are focussed on the detection of nucleic acids and homologues, particularly on the detection of mRNA, DNA, PNA, LNA and/or Morpholino.

In an embodiment of this invention the analysis uses mRNA including its precursor forms. In yet another embodiment the detection of methylation patterns and transcription factor footprints in gene regulatory regions such as promoter structures are used.

Moreover, these methods provide the option that high quality determinations can be done as multiplex assays in one reaction based on the high specificity of the reagent design and performance.

Moreover, these methods provide the option to determine the status of estrogen receptor and the status of progesterone receptor at the same time.

Another advantage is that the method requires only small amounts of biological sample.

In yet another preferred embodiment of the present invention it is provided that the expression level of the RNA transcripts and/or DNA amplification level of at least one gene encoding for a hormone receptor selected from the group comprising estrogen receptor, progesterone receptor and/or androgen receptor is determined by reverse transcriptase polymerase chain reaction (RT-PCR).

Especially, additional information based on the highly variable RNA expression of ESR1 as assessed by RT-PCR was found to enlarge the number of endocrine therapy responsive tumors determined compared to DNA analysis alone.

In yet another preferred embodiment of the present invention it is provided that determining the presence or absence of at least one single nucleotide polymorphism of a hormone receptor selected from the group comprising estrogen receptor, progesterone receptor and/or androgen receptor comprises contacting the biological sample with a reagent which specifically hybridizes to a single nucleotide polymorphism in any one of the nucleotide sequences of estrogen receptor selected from the group of dbSNP Submission_ID comprising rs2071454, rs28462265, rs3138774, rs9371556, rs9340770, rs9340771, rs867239, rs867240, rs34418993, rs41291045, rs9340772, rs872921, rs2077647, rs9340773, rs746432, rs17847065, rs17847076, rs9340802, rs4986934, rs1801132, rs17847067, rs9341068, rs9341069, rs34840398, rs2228480, rs9340799, and/or rs2234693 and/or the single nucleotide polymorphisms of progesterone receptor selected from the group group dbSNP Submission_ID comprising rs34720665, rs11571142, rs11571141, rs10895068, rs2008112, rs518162, rs11571140, rs11571139, rs11224606, rs11571138, rs625942, rs11571137, rs35974050, rs11571136, rs500760, rs11571252, rs2020880, rs2020878, rs1042839, rs1042838, rs11571222, rs2020874, rs2020876, rs11571179, rs11571152, rs11571151, rs11571150, rs1379130, rs11571149, rs11571148, rs11571147, rs3740753, rs35747049, rs34852313, rs11571146, rs10160588, rs10160726, rs36055552, rs11571145, rs11571144, rs3740754, and/or rs11571143 under stringent hybridization conditions, and detecting the formation of a hybridized duplex.

In another preferred embodiment of the present invention it is provided that determining the presence and/or absence of at least one single nucleotide polymorphism of a hormone receptor selected from the group comprising estrogen receptor, progesterone receptor and/or androgen receptor is carried out by a process selected from the group comprising allele-specific probe hybridization, allele-specific primer extension, allele-specific amplification, sequencing, 5' nuclease digestion, molecular beacon assay, oligonucleotide ligation assay, size analysis, and single-stranded conformation polymorphism. Preferred methods are described in Jaremko et al (2005), Hum Mutat 25:232; Saiki et al. (1986) Nature 324:163; Saiki et al (1989) Proc. Natl Acad. Sci USA 86:6230; Wallace et al. (1979) Nucl. Acids Res. 6:3543; Gibbs et al (1989) Nucleic Acids Res. 17:2437-2448; Prossner (1993) Tibtech 11:238; Newton et al. (1989) Nucl. Acids Res. 17:2503 or Gasparini et al (1992) Mol. Cell Probes 6:1 and in example 1c).

For analyzing single nucleotide polymorphisms (SNPs), it may be appropriate to use oligonucleotides specific for alternative SNP alleles. Such oligonucleotides which detect single nucleotide variations in target sequences may be referred to by such terms as "allele-specific oligonucleotides," "allele-specific probes," or "allele-specific primers." The design and use of allele-specific probes for analyzing polymorphisms is described in, e.g., EP 235 726 or WO 89/11548.

The method according to the invention has the advantage that it works on differently fixed paraffin embedded tissues, especially, preferably, formalin-fixed paraffin embedded tissues. In yet another preferred embodiment of the present invention, it is provided that the expression level of the RNA transcripts and/or DNA amplification level of at least one gene encoding for a hormone receptor selected from the group comprising estrogen receptor, progesterone receptor and/or androgen receptor, and/or the presence and/or absence of at least one single nucleotide polymorphism of estrogen receptor and/or progesterone receptor is determined in differently fixed paraffin embedded tissues, especially, preferably, formalin-fixed paraffin embedded tissues.

For this purpose, at least one fixative may used in a preferred embodiment which is selected from the group consisting of Neutral Buffered Formaline, Unbuffered Formaline, Ethanol, Acetone, Methanol, Methacarn, Carnoy's fixative, AFA-Fixative (Formaldehyde, Ethanol and acetic acid), glutaraldehyde, Pen-Fix (alcoholic formalin fixative), Glyo-Fixx (glyoxal-based fixative), Hope (Hepes-glutamic acid buffer mediated organic solvent fixative), and/or Zinc Formal-Fixx (Formaldehyde fixative which contains zinc).

In yet another preferred embodiment of the present invention, it is provided that the expression level of at least one of the said ligands or receptors is determined in serum, plasma or whole blood samples.

Routinely, in tumor diagnosis tissue samples are taken as biopsies form a patient and undergo diagnostic procedures. For this purpose, the samples are fixed and embedded in formaline and/or parrafine and are then examined with immunohistochemistry methods. The formaline treatment leads to the inactivation of enzymes, as for example the ubiquitous RNA-digesting enzymes (RNAses). Although the RNA is degraded and the integrity is affected over time, the relative quantification level of a target gene in comparison to a reference or housekeeper gene, remains unaffected.

However, the formaline treatment may lead to partial degradation of the individual mRNA molecules. The same applies for other fixatives, as for example mentioned in the above enumeration. For this reason, the current doctrine is that fixed tissue samples can not be used for the analysis of the transcriptome of said tissue.

For this reason, it is provided in a preferred embodiment of the present invention that after lysis, the samples are treated with silica-coated magnetic particles and a chaotropic salt, in order to purify the nucleic acids contained in said sample for further determination.

Collaborators of the inventors of the present invention have developed an approach which however allows successful purification of mRNA out of tissue samples fixed in such manner, and which is disclosed, among others, in WO03058649, WO2006136314A1 and DE10201084A1, the content of which is incorporated herein by reference.

Said method comprises the use of magnetic particles coated with silica (siO₂). The silica layer is closed and tight and is characterized by having an extremely small thickness on the scale of a few nanometers. These particles are produced by an improved method that leads to a product having a closed silica layer and thus entail a highly improved purity. The said method prevents an uncontrolled formation of aggregates and clusters of silicates on the magnetite surface whereby positively influencing the additional cited properties and biological applications. The said magnetic particles exhibit an optimized magnetization and suspension behavior as well as a very advantageous run-off behavior from plastic surfaces. These highly pure magnetic particles coated with silicon dioxide are used for isolating nucleic acids, including DNA and RNA, from cell and tissue samples, the separating out from a sample matrix ensuing by means of magnetic fields. These particles are particularly well-suited for the automatic purification of nucleic acids, mostly from biological body samples for the purpose of detecting them with different amplification methods.

The selective binding of these nucleic acids to the surface of said particles is due to the affinity of negatively charged nucleic acids to silica containing media in the presence of chaotropic salts like guanidiniumisothiocyanate. Said binding properties are known as the so called "boom principle". They are described in the European patent EP819696, the content of which is incorporated herein by reference.

The said approach is particularly useful for the purification of mRNA out of formaline and/or paraffine fixed tissue samples. In contrast to most other approaches, which leave very small fragments behind that are not suitable for later determination by PCR and/or hybridization technologies, the said approach creates mRNA fragments which are large enough to allow specific primer hybridzation and/or specific probe hybridization. A minimal size of at least 100 bp, more preferably 200 base pairs is needed for specific and robust detection of target gene expression. Moreover it is also necessary to not have too many inter-sample variations with regard to the size of the RNA fragments to guarantee comparability of gene expression results. Other issues of perturbance of expression data by sample preparation problems relate to the contamination level with DNA, which is lower compared to other bead based technologies.

The said approach thus allows a highly specific determination of the status of estrogen receptor and/or progesterone receptor with one of the above introduced methods, particularly with hybridization based methods, PCR based methods and/or array based methods, even in formaline and/or paraffine fixed tissue samples, and is thus extremely beneficial in the context of the present invention, as it allows the use of tissue samples fixed with formaline and/or paraffine, which are available in tissue banks and connected to clinical databases of sufficient follow-up to allow retrospective analysis.

An important aspect is that the inventors found that based on a magnetic bead technology, high quality RNA and DNA can be isolated and assessed by PCR technologies with more than 90% of the samples being analyzable. Even limited amounts such as 1 µm cuts of 1.5 mm diameter from tumor tissue were found to be sufficient for this purposes.

However the isolation method may alternatively also be silica column based with or without chaotropic agents.

Another important aspect is that the said approach allows the simultaneous determination of more than one analyte (multiplexing), and is thus ideally suited for the determination of the status of a hormone receptor selected from the group comprising estrogen receptor, progesterone receptor and/or androgen receptor in said sample, as provided by the method according to the present invention.

Another advantage is that thereby the expression level of a housekeeping gene can be determined simultaneously. By this approach one can derive a calibration factor in order to normalize the expression values of the target genes in samples which have different shares of tumor tissue and non tumor tissue.

In a preferred embodiment of the method of the invention the predicting includes determining a predictor value algorithmically.

In a yet preferred embodiments of the method of the invention it is provided that information on expression levels of the RNA transcripts of at least one gene encoding for a hormone receptor selected from the group comprising estrogen receptor, progesterone receptor and/or androgen receptor, and/or information on amplification level of at least one gene encoding for a hormone receptor selected from the group comprising estrogen receptor, progesterone receptor and/or androgen receptor, and/or information on the presence or absence of at least one single nucleotide polymorphism of at least one gene encoding for a hormone receptor selected from the group comprising estrogen receptor, progesterone receptor and/or androgen receptor are combined in a mathematical function that discriminates two or more patient outcome groups.

In a yet preferred embodiments of the method of the invention it is provided that information on expression levels of the RNA transcripts of at least one gene encoding for a hormone receptor selected from the group comprising estrogen receptor, progesterone receptor and/or androgen receptor, and/or information on amplification level of at least one gene encoding for a hormone receptor selected from the group comprising estrogen receptor, progesterone receptor and/or androgen receptor, and/or information on the presence or absence of at least one single nucleotide polymorphism of at least one gene encoding for a hormone receptor selected from the group comprising estrogen receptor, progesterone receptor and/or androgen receptor are combined in a mathematical function that assesses outcome in terms of a continuous score.

Preferably the mathematical function discriminates two or more patient outcome groups. In yet another preferred embodiment, the mathematical function assesses outcome in terms of a continuous score which may be discretized to obtain different risk groups.

In a preferred embodiment of the present invention said patient is a ESR1 positive breast cancer patient.

In a yet preferred embodiment of the present invention said neoplastic disease or cancer is selected from the group gynecologic cancers selected from the group comprising ovarian cancer, vulvar cancer, vaginal cancer, tubal cancer, endometrian cancer and/or cervical cancer.

In an especially preferred embodiment of the present invention said neoplastic disease or cancer is breast cancer. The method according to the invention may be used for the analysis of a wide variety of neoplastic cell growth and proliferation of the breast tissues including, but not limited to ductal carcinoma in situ, lobular carcinoma, colloid carcinoma, tubular carcinoma, medullary carcinoma, metaplastic carcinoma, intraductal carcinoma in situ, lobular carcinoma in situ and papillary carcinoma in situ.

In yet another preferred embodiment of the present invention, it is provided that said endocrine treatment is a hormonal treatment and/or antihormonal treatment.

Said endocrine treatment may comprises the administration of antagonists of estrogen binding to the estrogen receptor, estrogen reuptake inhibitors, selective estrogen receptor downregulators, or as inhibitors of estrogen biosynthesis, such as aromatase inhibitors.

Said endocrine treatment may include administration of hormones or hormone analogs, synthetic hormones or other drugs to the patient, e.g. tamoxiten or aromatases. In a preferred embodiment the said endocrine treatment comprises the administration of tamoxifen.

In other preferred embodiments said endocrine treatment may comprise the administration of an anti estrogen drug selected from the group comprising anastrozole, letrozole, exemestane, fulvestrant, toremifene, fulvestrant and megasterol acetate.

It is yet another embodiment of the present invention to provide a method for predicting the development of resistance to therapeutic intervention of a patient suffering from breast cancer and/or gynaecological cancers to a given treatment.

It is yet a preferred embodiment of the present invention to provide a method to stratify patients for systemic treatments other than chemotherapy in a neoadjuvant, adjuvant or palliative setting. In a preferred embodiment these alternative treatment options comprise antibody based or small molecule based treatment. However in a most preferred embodiment this relates to endocrine treatment options.

In yet another embodiment of the invention a method for correlating the clinical outcome of a patient suffering from or at risk of developing a neoplastic disease with the presence or non-presence of a defect in expression levels of the RNA transcripts of at least one gene encoding for a hormone receptor selected from the group comprising estrogen receptor, progesterone receptor and/or androgen receptor, and/or the DNA amplification level of at least one gene encoding for a hormone receptor selected from the group comprising estrogen receptor, progesterone receptor and/or androgen receptor, and/or the presence or absence of at least one single nucleotide polymorphism of a hormone receptor selected from the group comprising estrogen receptor, progesterone receptor and/or androgen receptor is provided, said method comprising the steps of:
a) obtaining a biological sample from said patient;
b) determining the status of at least one hormone receptor selected from the group comprising estrogen receptor, progesterone receptor and/or androgen receptor in said sample, using least two determinations selected from the group comprising:
   aa) determining the expression level of the RNA transcripts of at least one gene encoding for a hormone receptor selected from the group comprising estrogen receptor, progesterone receptor and/or androgen receptor, and/or
   bb) determining the DNA amplification level of at least one gene encoding for a hormone receptor selected from the group comprising estrogen receptor, progesterone receptor and/or androgen receptor, and/or
   cc) determining the presence or absence of at least one single nucleotide polymorphism of a hormone receptor selected from the group comprising estrogen receptor, progesterone receptor and/or androgen receptor, and
c) correlating the pattern of expression level(s) of aa), amplification level(s) of bb) and/or the single nucleotide polymorphism(s) of cc) determined in step b) with said patient's data, said data being selected from the group consisting of etiopathology data, clinical symptoms, anamnesis data and/or data concerning the therapeutic regimen.

The said method is particularly beneficial for epidemiological studies. These studies profit from the fact that large tissue databases exist comprising paraffin and/or formalin fixed tissue samples together with an extensive documentation of the patient's history, including etiopathology data, clinical symptoms, anamnesis data and/or data concerning the therapeutic regimen. The said methods advantageously allows for large scale studies.

In a further aspect, the present invention provides a method of selecting a therapy modality for a patient afflicted with a neoplastic disease, said method comprising the steps of:
a) obtaining a biological sample from said patient;
b) predicting from said sample, by the method according to any one of the aforementioned claims, therapeutic outcome for a plurality of individual modes of treatment; and
c) selecting a mode of treatment which is predicted to be successful in step b).

The invention also provides a method for adapting therapeutic regimen based on individualized risk assessment for a patient suffering from or at risk of developing a neoplastic disease, comprising the steps of:
a) obtaining a biological sample from said patient;
b) determining the status of at least one hormone receptor selected from the group comprising estrogen receptor, progesterone receptor and/or androgen receptor in said sample, using at least two determinations selected from the group comprising:
   aa) determining the expression level of the RNA transcripts of at least one gene encoding for a hormone receptor selected from the group comprising estrogen receptor, progesterone receptor and/or androgen receptor, and/or
   bb) determining the DNA amplification level of at least one gene encoding for a hormone receptor selected from the group comprising estrogen receptor, progesterone receptor and/or androgen receptor, and/or
   cc) determining the presence or absence of at least one single nucleotide polymorphism of at least one gene encoding for a hormone receptor selected from the group comprising estrogen receptor, progesterone receptor and/or androgen receptor;
c) comparing the pattern of expression level(s) of aa), amplification level(s) of bb) and/or the single nucleotide polymorphism(s) of cc) determined in step b) with one or several reference pattern(s) of expression and/or amplification levels and/or single nucleotide polymorphisms; and
d) implementing therapeutic regimen targeting a hormone receptor selected from the group comprising estrogen receptor, progesterone receptor and/or androgen receptor in said patient from the outcome of the comparison in step c).

The inventors suggest, for the first time, to use the expression level of a gene encoding for a hormone receptor selected from the group comprising estrogen receptor, progesterone receptor and/or androgen receptor, especially ESR1, for the decision whether or not endocrine treatment should be included as a treatment option.

In this regard, the detection of hormone receptor status selected from the group comprising estrogen receptor, progesterone receptor and/or androgen receptor status by a combinatorial analysis of hormone receptor status selected from the group comprising estrogen receptor, progesterone receptor and/or androgen receptor status on at least two different levels, of the expression level of the RNA transcripts, the DNA amplification level, and/or the presence or absence of at least one single nucleotide polymorphism enables to identify tumors that overexpress ESR1 and/or PGR that cannot be resolved by immunohistochemical techniques.

In another preferred embodiment of the present invention, a kit useful for carrying out a method of the invention is provided, comprising at least a pair of gene specific primers and/or probes each having a sequence sufficiently complementary to at least one gene or gene fragments or genomic nucleic acid sequence encoding for a at least one marker selected from the group comprising a hormone receptor selected from the group comprising estrogen receptor, progesterone receptor and/or androgen receptor for quantifying the expression of said at least one gene or gene fragment or genomic nucleic acid sequence, and/or their fractions, variants, homologues, derivatives, fragments, complements, hybridizing counterparts, or molecules sharing a sequence identity of at least 70%, preferably 95 %.

Said kit may, in another preferred embodiment, comprise specific primers and/or probes each having a sequence sufficiently complementary to at least one of the single nucleotide polymorphisms of estrogen receptor selected from the group of dbSNP Submission_ID comprising rs2071454, rs28462265, rs3138774, rs9371556, rs9340770, rs9340771, rs867239, rs867240, rs34418993, rs41291045, rs9340772, rs872921, rs2077647, rs9340773, rs746432, rs17847065, rs17847076, rs9340802, rs4986934, rs1801132, rs17847067, rs9341068, rs9341069, rs34840398, rs2228480, rs9340799, and/or rs2234693 and/or the single nucleotide polymorphisms of progesterone receptor selected from the group group dbSNP Submission_ID comprising rs34720665, rs11571142, rs11571141, rs10895068, rs2008112, rs518162, rs11571140, rs11571139, rs11224606, rs11571138, rs625942, rs11571137, rs35974050, rs11571136, rs500760, rs11571252, rs2020880, rs2020878, rs1042839, rs1042838, rs11571222, rs2020874, rs2020876, rs11571179, rs11571152, rs11571151, rs11571150, rs1379130, rs11571149, rs11571148, rs11571147, rs3740753, rs35747049, rs34852313, rs11571146, rs10160588, rs10160726, rs36055552, rs11571145, rs11571144, rs3740754, and/or rs11571143, and/or their fractions, variants, homologues, derivatives, fragments, complements, hybridizing counterparts, or molecules sharing a sequence identity of at least 70%, preferably 95 %.

These nucleic acids can be used either as primers for a polymerase chain reaction protocol, or as detectable probes for monitoring the said process.

Furthermore it is provided that the said nucleic acid and homologues is selected from the group consisting of DNA, RNA, PNA, LNA and/or Morpholino. The nucleic acid may, in a preferred embodiment, be labelled with at least one detectable marker. This feature is applicable particularly for those nucleic acids which serve as detectable probes for monitoring the polymerase chain reaction process.

Such detectable markers may for example comprise at least one label selected from the group consisting of fluorescent molecules, luminescent molecules, radioactive molecules, enzymatic molecules and/or quenching molecules.

In a particularly preferred embodiment, the said detectable probes are labeled with a fluorescent marker at one end and a quencher of fluorescence at the opposite end of the probe. The close proximity of the reporter to the quencher prevents detection of its fluorescence; breakdown of the probe by the 5' to 3' exonuclease activity of the taq polymerase breaks the reporter-quencher proximity and thus allows unquenched emission of fluorescence, which can be detected. An increase in the product targeted by the reporter probe at each PCR cycle therefore causes a proportional increase in fluorescence due to the breakdown of the probe and release of the reporter.

### Disclaimer

To provide a comprehensive disclosure without unduly lengthening the specification, the applicant hereby incorporates by reference each of the patents and patent applications referenced above.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. The invention's scope is defined in the following claims and the equivalents thereto. Furthermore, reference signs used in the description and claims do not limit the scope of the invention as claimed.

### Brief description of the examples

Additional details, features, characteristics and advantages of the object of the invention are disclosed in the subclaims, and the following description of the respective figures and examples, which, in an exemplary fashion, show preferred embodiments of the present invention. However, these examples should by no means be understood as to limit the scope of the invention.

### Example 1

Combination of biomarker information of quantification of gene copy numbers, RNA expression, and DNA polymorphisms for prediction of endocrine treatment efficacy

1a: Quantification of gene copy numbers by quantitative PCR Usually, one to three paraffin-embedded tissue sections that are 5 µm thick are used to obtain genomic DNA from formalin-fixed paraffin-embedded samples. The DNA can be extracted by different commercially available methods like DEXPAT™ kit (TaKaRa), the QIAamp^{®} DNA Mini Kits or QIAamp^{®} RNA FFPE Kits (Qiagen) (copurifies DNA) according to the manufacturers protocols. Eluates can be quantified by the Picogreen RNA Quantitation Assay (Molecular Probes/Invitrogen).

For a detailed analysis of gene copy number by quantitative PCR methods, primers are used flanking the genomic region of interest and a fluorescent labelled probe hybridizing in-between. Using the PRISM 7700 or 7900 Sequence Detection System of Applied Biosystems (Perkin Elmer, Foster City, CA, USA) with the technique of a fluorogenic probe, consisting of an oligonucleotide labelled with both a fluorescent reporter dye and a quencher dye, such a measurement can be performed. Amplification of the probe-specific product causes cleavage of the probe, generating an increase in reporter fluorescence. Primers and probes were selected using the Primer Express® software v2.0 (Applied Biosystems) according to manufacturers instructions and can be localized in introns to guarantee DNA-specificity. Predefined primer and probes for the genes can also be obtained from suppliers e.g. Applied Biosystems. All primer pairs were checked for specificity by conventional PCR reactions and gel electrophoresis.

To measure the gene copy number of the ESR1 or PGR genes within the patient sample eluate, the respective primer/probes were prepared by mixing 25 µl of the 100 µM stock solution "Forward Primer", 25 µl of the 100 µM stock solution "Reverse Primer" with 12.5 µl of the 100 µM stock solution Taq Man Probe (Quencher Tamra) and adjusted to 500 µl with aqua dest. For each reaction, 1.25 µl eluate or an equivalent of about 2 ng DNA were mixed with 8.75 µl nuclease-free water and added to one well of a 96 Well-Optical Reaction Plate (Applied Biosystems Part No. 4306737). 1.5 µl Primer/Probe mix, 12.5µl Taq Man^{®} Universal-PCR Mix (2x conc.) (Applied Biosystems (Part No. 4318157)) and 1 µl Water were then added. The 96 well plates were closed with 8 Caps/Strips (Applied Biosystems (Part Number 4323032)). Measurements of the PCR reaction are done according to the instructions of the manufacturer with a TaqMan^{®} 7900 HT from Applied Biosystems (No. 20114) under appropriate conditions (2 min. 50°C, 10 min. 95°C, 0.15 min. 95°C, 1 min. 60°C; 40 cycles). SoftwareSDS 2.0 from Applied Biosystems was used according to the respective instructions. CT-values were then further analyzed with appropriate software (Microsoft Excel^{™})_{.}

Extrachromosomal reference genes for assessment of gene copy amplification included GAPDH for chromosome 12 and MMP28 for chromosome 17 and TRIM27 for chromosome 6(p-region) and SNX3 for chromosome 6 (q-region) (see Table 1). However, other genes not disclosed in this invention can be used as reference gene for copy number analysis in addition or alternatively to those disclosed. The reference genes shall be not affected by chromosomal alterations such as amplifications and deletions. Reference genes may additionally be measured in simplex, multiplex or in metaplex which is the simultaneous measurement of multiple targets with multiple primer/probes and identical fluorophors. Additionally, measurement of optical density (OD) may be used as a reference in addition or alternatively to reference genes. As gene copy numbers of non-amplified genes can be increased in neoplastic lesions due to genomic imbalances such as aneuploidy or polyploidy, each measurement of the ESR or PGR genes was correlated to multiple reference genes to minimize the influence of genomic imbalances on the relative copy number calculation. Moreover, minor systematic errors occurring due to differences in the performance of individual primer/probe pairs were minimized by determining primer/probe performances in control tissues, i.e. non-neoplastic tissues from healthy controls, and euploid control cell lines, e.g. HS68, ATCC #CRL1635. In addition, synthetic targets were spiked into some reactions that consisted of the target region of the PCR forward and reverse primers of the gene to be normalized, but in between consisted of a synthetic probe hybridization region different from the original probe region of the target gene to be normalized. This allowed internal standardization of each individual quantitative PCR (qPCR) reaction by multiplex PCR. The calculated performance differences were used as a filter for the measurements within the target tissues, i.e. primer/probe performance differences of each individual gene as depicted in the control cells with defined copy numbers or copy number ratio to a reference and tissues were subtracted from each individual gene measurement performed in the target tissue. Thereafter, the individual, filtered CT values were normalized to the reference. Differences between the CT values of the quantitative PCR reactions of the ESR1 or PGR genes and the reference remaining after filtering the primer/probe performance differences were determined and transformed into "copy numbers per cell". This was done by subtracting the CT values of the target genes from the CT values of the reference. The resulting •CT values were then transformed in gene copy numbers, with the •CT value of the reference (•CT=0) being defined as "2 copies per cell", by the following formula: 2*(2^(-•CT)). All the calculations were done using standard software (Microsoft Excel^{™})_{.}

1b: Quantification of RNA expression by quantitative RT-PCR RNA was isolated from paraffin-embedded, formalin-fixed tissues (= FFPE tissues). Those skilled in the art are able to perform RNA extraction procedures. For example, total RNA from a 5 to 10 µm cut of FFPE tumor tissue can be extracted using the High Pure RNA Paraffin Kit (Roche, Basel, Switzerland) or QIAamp^{®} RNA FFPE Kit, quantified by the Ribogreen RNA Quantitation Assay (Molecular Probes/Invitrogen) and quantified by real-time fluorescence RT-PCR of RPL37A.

To measure the RNA expression level of the ESR or PGR genes, in general 0.5 to 2 ng RNA of each qualified RNA extraction was assayed by qRT-PCR as described below. For a detailed analysis of gene expression by quantitative PCR methods, one will utilize primers flanking the genomic region of interest and a fluorescent labelled probe hybridizing in-between. Using a MX4000 device of Stratagene using the technique of a fluorogenic probe, consisting of an oligonucleotide labeled with both a fluorescent reporter dye and a quencher dye, one can perform such an expression measurement. Amplification of the probe-specific product causes cleavage of the probe, generating an increase in reporter fluorescence. Primers and probes were selected using the Primer Express software (Version 2.0) and preferably localized across exon/exon boundary and large intervening non-transcribed sequences (> 800 bp) to guarantee RNA-specificity or with in the 3' region of the coding sequence or in the 3' untranslated region. Primer design and selection of an appropriate target region is well known to those skilled in the art. Predefined primer and probes for the genes listed in Table 1 can also be obtained from suppliers e.g. Applied Biosystems. All primer pairs were checked for specificity by conventional PCR reactions and gel electrophoresis.

To standardize the amount of sample RNA, GAPDH, RPL37A, RPL9 and CD63 were selected as references, since they were not differentially regulated in the samples analyzed. To perform a one step RT-PCR expression analysis of genes 0.5 to 2 ng eluate were added to 20 µl of MasterMix. This consists of the following components: 400 nM forward primer, 400 nM reverse primer, 200 nM Taqman^{®}- primer (FAM/TAMRA labeled), 1 x buffer A, 5 mM MgCl2; 1.2 mM dNTPs, 8 U RNase inhibitor, 20 U MuLV Reverse Transcriptase, 1.25 U Taq Gold (all components from Applied Biosystems). The PCR program was as follows: 30 minutes at 45°C, 10 minutes at 95°C, 40 cycles of 15 seconds at 96°C, 60 seconds at 63°C and 30 seconds at 72°C).

Prior to the measurement of so far unclassified biological samples control experiments with e.g. cell lines, healthy control samples, samples of defined therapy response were used for standardization of the experimental conditions.

For determining CT values SoftwareSDS 2.0 from Applied Biosystems were used according to the respective instructions. CT-values were then further analyzed with appropriate software (Microsoft Excel^{™})

The PCR efficiencies of the target and the reference must be approximately equal which is a prerequisite for the relative quantification of gene expression by the comparative •CT method, known to those skilled in the art. In general CT values were normalized by subtracting the CT value of the reference gene or the mean CT value of all reference genes from the CT score of the target gene (•CT). RNA expression values were calculated as 40-•CT scores, which correlate proportionally to the mRNA expression level of the target gene. Using the calculation 2^{(40-•CT)} , , the logarithmic CT values can be transformed to linear "copy-like" expression values for each individual target gene.

1c: Detection of DNA polymorphisms and allelic differences Examples of techniques for detecting differences of at least one nucleotide between 2 nucleic acids include, but are not limited to, selective oligonucleotide hybridization, selective amplification, or selective primer extension. Commercial MALDI/TOF MS platforms from companies like Sequenom (www.sequenom.com) also offer full protocols for single nucleotide polymorphism determination. A comparable MALDI/TOF MS method is also described in Jaremko et al (2005), Hum Mutat 25:232.

In an exemplary implementation, oligonucleotide probes may be prepared in which the known polymorphic nucleotide is placed centrally (allele-specific probes) and then hybridized to target DNA under conditions which permit hybridization only if a perfect match is found (Saiki et al. (1986) Nature 324:163); Saiki et al (1989) Proc. Natl Acad. Sci USA 86:6230; and Wallace et al. (1979) Nucl. Acids Res. 6:3543). Such allele specific oligonucleotide hybridization techniques may be used for the simultaneous detection of several nucleotide changes in different polymorphic regions of gene. For example, oligonucleotides having nucleotide sequences of specific allelic variants are attached to a hybridizing membrane and this membrane is then hybridized with labeled sample nucleic acid. Analysis of the hybridization signal will then reveal the identity of the nucleotides of the sample nucleic acid.

In another exemplary implementation, allele specific amplification technology which depends on selective PCR amplification may be used. Oligonucleotides used as primers for specific amplification may carry the allelic variant of interest in the center of the molecule, so that amplification depends on differential hybridization (Gibbs et al (1989) Nucleic Acids Res. 17:2437-2448) or at the extreme 3' end of one primer where, under appropriate conditions, mismatch can prevent, or reduce polymerase extension (Prossner (1993) Tibtech 11:238; Newton et al. (1989) Nucl. Acids Res. 17:2503). This technique is also termed "PROBE" for Probe Oligo Base Extension.

In addition it may be desirable to introduce a novel restriction site in the region of the mutation to create cleavage-based detection (Gasparini et al (1992) Mol. Cell Probes 6:1). Normally 1 µl of eluate from the DNA extract or an equivalent of 2 ng was used in a 10 or 20 µl PCR reaction for SNP analysis.

1d: Combination of biomarker information of quantification of gene copy numbers, RNA expression, and DNA polymorphisms of 1a, 1b and 1c for prediction of endocrine treatment efficacy

The above described single marker measurements of target gene DNA copy numbers, target gene RNA copy number and the SNPs of both alleles in one gene position, was combined for classification of a patients clinical response or outcome under endocrine with either tamoxifen or aromatase inhibitor treatment.

By way of illustration and not by way of limitation, a breast cancer patient tumor sample with an ESR DNA copy number > 2 copies/cell (= amplification) and an ESR1 RNA expression level above a cut-off of the median expression (4300 copies/cell) of the RNA expression in the reference population (= overexpression) and homozygocity for Adenin in the ESR1 exon coding SNP rs9340773 (= Serin for codon usage) was classified as a responder, e.g. no distant metastasis in 5 years under endocrine treatment, e.g. tamoxifen.

In another breast cancer patient with an ESR1 DNA copy number > 2 copies/cell (= amplification) and an ESR RNA expression level above a cut-off of the median expression (........copies/cell) of the RNA expression in the reference population (= overexpression) and homozygocity for Guanin in the ESR1 exon coding SNP rs9340773 (= Glycin for codon usage) was classified as a non-responder, e.g. distant metastasis in 5 years under endocrine treatment, e.g. tamoxifen.

In a third breast cancer patient where both the ESR1 DNA copy number or the RNA expression levels were below the above cutoffs, the patient was classified and predicted as a non-responder, e.g. distant metastasis in 5 years under endocrine treatment, e.g. tamoxifen. This classification was independent of any detected SNP in the ESR1, PGR or tamoxifen related metabolisms genes.

In a fourth breast cancer patient where the ESR1 DNA copy number is below 2 copies/cell (= no amplification) and an ESR1 RNA expression level is above a cut-off of 3rd quartile of the RNA expression (10000 copies/cell) in the reference population (= overexpression) and homozygocity for Adenin in the ESR1 exon coding SNP rs9340773 (= Serin for codon usage) was classified as a responder, e.g. no distant metastasis in 5 years under endocrine treatment, e.g. tamoxifen.

These examples show that a combination of biomarker information including the quantification of gene copy numbers, RNA expression values, and DNA single nucleotide polymorphisms provides the clinical useful information for a good prediction for endocrine treatment efficacy.

**Table 1: single nucleotide polymorphisms of estrogen receptor in accordance with the present invention**

| Gene_ Symbol [A] | Ref. Se quences [A] | dbSNP Submission_ID | Region | Contig position | mRNA position |
|---|---|---|---|---|---|
| ESR1 | NM_000125.2 | rs2071454 | 5' near gene | 56231253 | |
| ESR1 | NM_000125.2 | rs28462265 | 5' near gene | 56231534 | |
| ESR1 | NM_000125.2 | rs3138774 | 5' near gene | 56232091 | |
| ESR1 | NM_000125.2 | rs9371556 | 5' near gene | 56232094 | |
| ESR1 | NM_000125.2 | rs9340770 | 5' near gene | 56232531 | |
| ESR1 | NM_000125.2 | rs9340771 | 5' near gene | 56232984 | |
| ESR1 | NM_000125.2 | rs867239 | 5' near gene | 56233060 | |
| ESR1 | NM_000125.2 | rs867240 | 5' near gene | 56233071 | |
| ESR1 | NM 000125.2 | rs34418993 | 5' UTR | 56233122 | 8 |
| ESR1 | NM 000125.2 | rs41291045 | 5' UTR | 56233299 | 185 |
| ESR1 | NM 000125.2 | rs9340772 | 5' UTR | 56233307 | 193 |
| ESR1 | NM 000125.2 | rs872921 | 5' UTR | 56233368 | 254 |
| ESR1 | NM 000125.2 | rs2077647 | exon 1 | 56233506 | 392 |
| ESR1 | NM 000125.2 | rs9340773 | exon 1 | 56233705 | 591 |
| ESR1 | NM 000125.2 | rs746432 | exon 1 | 56233737 | 623 |
| ESR1 | NM 000125.2 | rs17847065 | exon 1 | 56233913 | 799 |
| ESR1 | NM 000125.2 | rs17847076 | exon 2 | 56268210 | 864 |
| ESR1 | NM 000125.2 | rs9340802 | exon 2 | 56268212 | 866 |
| ESR1 | NM 000125.2 | rs4986934 | exon 3 | 56306304 | 1091 |
| ESR1 | NM 000125.2 | rs1801132 | exon 4 | 56369951 | 1337 |
| ESR1 | NM 000125.2 | rs17847067 | exon 5 | 56437260 | 1499 |
| ESR1 | NM 000125.2 | rs9341068 | exon 8 | 56524416 | 2036 |
| ESR1 | NM 000125.2 | rs9341069 | exon 8 | 56524455 | 2075 |
| ESR1 | NM 000125.2 | rs34840398 | exon 8 | 56524503 | 2123 |
| ESR1 | NM 000125.2 | rs2228480 | exon 8 | 56524524 | 2144 |
| ESR1 | NM 000125.2 | rs9340799 | intron | 56267810 | |
| ESR1 | NM 000125.2 | rs2234693 | intron | 56267764 | |

In tables 1 and 2, the third column is labelled "dbSNP Submission_ID". The values in this column are SNP identification numbers according to the dbSNP database established and maintained by NCBI of the US National Library of Medicine at the US National Institute of Health. The NCBI dbSNP database is publicly accessible over the NCBI server and information can be easily viewed by searching the database using the rsID numbers provided. Sequences for given dbSNP_rsID numbers are found at: http://www.ncbi.nlm.nih.gov/SNP/.

The terms "Ref. Sequences" relates to databases in which the respective proteins are listed under the given access number. These databases can be accessed over the NCBI server.

**Table 2: single nucleotide polymorphisms of progesterone receptor used in accordance with the present invention**

| Gene_Symbol | Ref. Se quences | dbSNP Submission_ID | Region | Contig position | mRNA position |
|---|---|---|---|---|---|
| PGR | NM 000926.4 | rs34720665 | 5' UTR | 4562255 | 1417 |
| PGR | NM 000926.4 | rs11571142 | 5' UTR | 4562490 | 1182 |
| PGR | NM 000926.4 | rs11571141 | 5' UTR | 4562494 | 1178 |
| PGR | NM 000926.4 | rs10895068 | 5' UTR | 4562630 | 1042 |
| PGR | NM 000926.4 | rs2008112 | 5' UTR | 4562786 | 886 |
| PGR | NM 000926.4 | rs518162 | 5' UTR | 4562917 | 755 |
| PGR | NM 000926.4 | rs11571140 | 5' UTR | 4563243 | 429 |
| PGR | NM 000926.4 | rs11571139 | 5' UTR | 4563372 | 300 |
| PGR | NM 000926.4 | rs11224606 | 5' UTR | 4563455 | 217 |
| PGR | NM 000926.4 | rs11571138 | 5' UTR | 4563563 | 109 |
| PGR | NM_000926.4 | rs625942 | 5' near gene | 4563743 | |
| PGR | NM_000926.4 | rs11571137 | 5' near gene | 4563810 | |
| PGR | NM_000926.4 | rs35974050 | 5' near gene | 4563957 | |
| PGR | NM_000926.4 | rs11571136 | 5' near gene | 4564106 | |
| PGR | NM 000926.4 | rs500760 | exon 8 | 4472407 | 4112 |
| PGR | NM 000926.4 | rs11571252 | exon 7 | 4475134 | 4058 |
| PGR | NM 000926.4 | rs2020880 | exon 7 | 4475144 | 4048 |
| PGR | NM 000926.4 | rs2020878 | exon 7 | 4475152 | 4040 |
| PGR | NM 000926.4 | rs1042839 | exon 5 | 4484618 | 3764 |
| PGR | NM 000926.4 | rs1042838 | exon 4 | 4495828 | 3432 |
| PGR | NM 000926.4 | rs11571222 | exon 4 | 4495855 | 3405 |
| PGR | NM 000926.4 | rs2020874 | exon 3 | 4524939 | 3328 |
| PGR | NM 000926.4 | rs2020876 | exon 3 | 4524944 | 3323 |
| PGR | NM 000926.4 | rs11571179 | exon 3 | 4524983 | 3284 |
| PGR | NM 000926.4 | rs11571152 | exon 1 | 4560611 | 3061 |
| PGR | NM 000926.4 | rs11571151 | exon 1 | 4560633 | 3039 |
| PGR | NM 000926.4 | rs11571150 | exon 1 | 4560888 | 2784 |
| PGR | NM 000926.4 | rs1379130 | exon 1 | 4561039 | 2633 |
| PGR | NM 000926.4 | rs11571149 | exon 1 | 4561084 | 2588 |
| PGR | NM 000926.4 | rs11571148 | exon 1 | 4561163 | 2509 |
| PGR | NM 000926.4 | rs11571147 | exon 1 | 4561178 | 2494 |
| PGR | NM 000926.4 | rs3740753 | exon 1 | 4561187 | 2485 |
| PGR | NM 000926.4 | rs35747049 | exon 1 | 4561198 | 2474 |
| PGR | NM 000926.4 | rs34852313 | exon 1 | 4561207 | 2465 |
| PGR | NM 000926.4 | rs11571146 | exon 1 | 4561316 | 2356 |
| PGR | NM 000926.4 | rs10160588 | exon 1 | 4561429 | 2243 |
| PGR | NM 000926.4 | rs10160726 | exon 1 | 4561606 | 2066 |
| PGR | NM 000926.4 | rs36055552 | exon 1 | 4561657 | 2015 |
| PGR | NM 000926.4 | rs11571145 | exon 1 | 4561661 | 2011 |
| PGR | NM 000926.4 | rs11571144 | exon 1 | 4561859 | 1813 |
| PGR | NM 000926.4 | rs3740754 | exon 1 | 4562049 | 1623 |
| PGR | NM 000926.4 | rs11571143 | exon 1 | 4562070 | 1602 |

**Table 3: Genes of interest**

| Gene Symbol [A] | Ref. Sequences Description [A] | Ref. Sequences [A] | Unigene ID [A] |
|---|---|---|---|
| ESR1 | Estrogen receptor | NM 000125.2 | Hs.208124 |
| PGR | Progesterone receptor | NM 000926.4 | Hs.368072 |
| | | | Hs.525392; |
| ESR2 | Estrogen receptor | NM 001040276+1 | HS660607 |
| | | | Hs.525392; |
| ESR2 | Estrogen receptor | NM 001040276+1 | HS660607 |
| ESR2 | Estrogen receptor | NM 001437+2 | Hs.525392; HS660607 |
| AR | Androgen receptor | NM 000044.2 | Hs.496240 |
| AR | Androgen receptor | NM 001011645.1 | Hs.496240 |
| TRIM27 | tripartite motif-containing 27 | NM 006510.4 | Hs.440382 |
| SNX3 | sorting nexin 3 | NM 003795.3 | Hs.12102 |

The terms "Ref. Sequences" and "Unigene_ID" relate to databases in which the respective proteins are listed under the given access number. These databases can be accessed over the NCBI server.

### References

1 Saiki et al. (1986) Nature 324:163)
2 Saiki et al (1989) Proc. Natl Acad. Sci USA 86:6230
3 Wallace et al. (1979) Nucl. Acids Res. 6:3543
4 Gibbs et al (1989) Nucleic Acids Res. 17:2437-2448)
5 Prossner (1993) Tibtech 11:238
6 Newton et al. (1989) Nucl. Acids Res. 17:2503
7 Gasparini et al (1992) Mol. Cell Probes 6:1
8 Jaremko et al (2005)Hum Mutat 25:232

## Claims

1. A method for predicting a clinical response of a patient suffering from or at risk of developing a neoplastic disease to a given mode of treatment by determination the status of at least one hormone receptor selected from the group comprising estrogen receptor, progesterone receptor and/or androgen receptor, said method comprising the steps of:
a) obtaining a biological sample from said patient;
b) determining the status of at least one hormone receptor selected from the group comprising estrogen receptor, progesterone receptor and/or androgen receptor in said sample, using at least two determinations selected from the group comprising:
aa) determining the expression level of the RNA transcripts of at least one gene encoding for a hormone receptor selected from the group comprising estrogen receptor, progesterone receptor and/or androgen receptor, and/or
bb) determining the DNA amplification level of at least one gene encoding for a hormone receptor selected from the group comprising estrogen receptor, progesterone receptor and/or androgen receptor, and/or
cc) determining the presence or absence of at least one single nucleotide polymorphism of at least one gene encoding for a hormone receptor selected from the group comprising estrogen receptor, progesterone receptor and/or androgen receptor;
c) comparing the pattern of expression level(s) of aa), amplification level(s) of bb) and/or the single nucleotide polymorphism(s) of cc) determined in step b) with one or several reference pattern(s) of expression and/or amplification levels and/or single nucleotide polymorphisms; and
d) predicting therapeutic outcome for said given mode of treatment in said patient from the outcome of the comparison in step c).

2. The method according to claim 1 **characterized in that** said given mode of treatment is endocrine treatment.

3. The method according to claim 1 or 2, **characterized in that** the gene encoding for estrogen receptor which is determined is ESR1 and/or ESR2.

4. The method according to any one of the aforementioned claims, **characterized in that** the single nucleotide polymorphism of estrogen receptor is selected from the group of dbSNP Submission_ID comprising rs2071454, rs28462265, rs3138774, rs9371556, rs9340770, rs9340771, rs867239, rs867240, rs34418993, rs41291045, rs9340772, rs872921, rs2077647, rs9340773, rs746432, rs17847065, rs17847076, rs9340802, rs4986934, rs1801132, rs17847067, rs9341068, rs9341069, rs34840398, rs2228480, rs9340799, and/or rs2234693.

5. The method according to any one of the aforementioned claims, **characterized in that** the single nucleotide polymorphisms of progesterone receptor are selected from the group of dbSNP Submission_ID comprising rs34720665, rs11571142, rs11571141, rs10895068, rs2008112, rs518162, rs11571140, rs11571139, rs11224606, rs11571138, rs625942, rs11571137, rs35974050, rs11571136, rs500760, rs11571252, rs2020880, rs2020878, rs1042839, rs1042838, rs11571222, rs2020874, rs2020876, rs11571179, rs11571152, rs11571151, rs11571150, rs1379130, rs11571149, rs11571148, rs11571147, rs3740753, rs35747049, rs34852313, rs11571146, rs10160588, rs10160726, rs36055552, rs11571145, rs11571144, rs3740754, and/or rs11571143.

6. The method according to any one of the aforementioned claims, wherein upregulated expression of the RNA transcripts of at least one gene encoding for a hormone receptor selected from the group comprising estrogen receptor, progesterone receptor and/or androgen receptor, and/or of the DNA amplification level of a hormone receptor selected from the group comprising estrogen receptor, progesterone receptor and/or androgen receptor, and/or the determining of the presence or absence of at least one single nucleotide polymorphism of a hormone receptor selected from the group comprising estrogen receptor, progesterone receptor and/or androgen receptor determined in step b) is indicative of a prediction as regards therapeutic outcome for endocrine treatment.

7. The method according to any one of the aforementioned claims, **characterized in that** in step b) additionally the presence or absence of at least one single nucleotide polymorphism of at least one tamoxifen metabolism gene is determined.

8. The method according to any one of the aforementioned claims, **characterized in that** the tamoxifen metabolism genes are selected from the group comprising CYP2D6, SULT1A1, UGT2B15, CYP2C19, CYP3A5, CYP2B6, and/or CYP2C9.

9. The method according to any one of the aforementioned claims, **characterized in that** in step b) the status of estrogen receptor is determined comprising the steps of:
aa) determining the RNA expression level of estrogen receptor, and
bb) determining the DNA amplification level of estrogen receptor, and
cc) determining the presence or absence of at least one single nucleotide polymorphism of estrogen receptor.

10. The method according to any one of the aforementioned claims, wherein the expression level and/or amplification level and/or presence or absence of at least one single nucleotide polymorphism determined in step b) are determined by
a) a hybridization based method;
b) a PCR based method;
c) a method based on the electrochemical detection of particular molecules, and/or by
d) an array based method.

11. The method according to any one of the aforementioned claims, **characterized in that** the expression level of the RNA transcripts and/or DNA amplification level of at least one gene encoding for a hormone receptor selected from the group comprising estrogen receptor, progesterone receptor and/or androgen receptor is determined by reverse transcriptase polymerase chain reaction.

12. The method according to any one of the aforementioned claims, **characterized in that** determining the presence or absence of at least one single nucleotide polymorphism of a hormone receptor selected from the group comprising estrogen receptor, progesterone receptor and/or androgen receptor comprises contacting the biological sample with a reagent which specifically hybridizes to a single nucleotide polymorphism of estrogen receptor selected from the group of dbSNP Submission_ID comprising rs2071454, rs28462265, rs3138774, rs9371556, rs9340770, rs9340771, rs867239, rs867240, rs34418993, rs41291045, rs9340772, rs872921, rs2077647, rs9340773, rs746432, rs17847065, rs17847076, rs9340802, rs4986934, rs1801132, rs17847067, rs9341068, rs9341069, rs34840398, rs2228480, rs9340799, and/or rs2234693 and/or the single nucleotide polymorphisms of progesterone receptor selected from the group group dbSNP Submission_ID comprising rs34720665, rs11571142, rs11571141, rs10895068, rs2008112, rs518162, rs11571140, rs11571139, rs11224606, rs11571138, rs625942, rs11571137, rs35974050, rs11571136, rs500760, rs11571252, rs2020880, rs2020878, rs1042839, rs1042838, rs11571222, rs2020874, rs2020876, rs11571179, rs11571152, rs11571151, rs11571150, rs1379130, rs11571149, rs11571148, rs11571147, rs3740753, rs35747049, rs34852313, rs11571146, rs10160588, rs10160726, rs36055552, rs11571145, rs11571144, rs3740754, and/or rs11571143 under stringent hybridization conditions, and detecting the formation of a hybridized duplex.

13. The method according to any one of the aforementioned claims, **characterized in that** determining the presence or absence of at least one single nucleotide polymorphism of a hormone receptor selected from the group comprising estrogen receptor, progesterone receptor and/or androgen receptor is carried out by a process selected from the group comprising allele-specific probe hybridization, allele-specific primer extension, allele-specific amplification, sequencing, 5' nuclease digestion, molecular beacon assay, oligonucleotide ligation assay, size analysis, and single-stranded conformation polymorphism.

14. The method according to any one of the aforementioned claims, **characterized in that** the expression level of the RNA transcripts and/or DNA amplification level of at least one gene encoding for a hormone receptor selected from the group comprising estrogen receptor, progesterone receptor and/or androgen receptor, and/or the presence or absence of at least one single nucleotide polymorphism of a hormone receptor selected from the group comprising estrogen receptor, progesterone receptor and/or androgen receptor is determined in formalin and/or paraffin fixed tissue samples.

15. The method according to any one of the aforementioned claims, wherein, after lysis, the sample is treated with silica-coated magnetic particles and a chaotropic salt, for purification of the nucleic acids contained in said sample for further determination.

16. The method according to any one of the aforementioned claims **characterized in that** said neoplastic disease or cancer is selected from the group comprising breast cancer and gynecologic cancers selected from the group comprising ovarian cancer, vulvar cancer, vaginal cancer, tubal cancer, endometrian cancer and/or cervical cancer.

17. The method according to any one of the aforementioned claims **characterized in that** said neoplastic disease or cancer is breast cancer.

18. The method according to any one of the aforementioned claims **characterized in that** said patient is a ESR1 positive breast cancer patient.

19. The method according to any one of the aforementioned claims, **characterized in that** said endocrine treatment is a hormonal treatment and/or antihormonal treatment.

20. The method according to any one of the aforementioned claims, **characterized in that** said endocrine treatment comprises the administration of tamoxifen.

21. The method according to any one of the aforementioned claims, **characterized in that** said endocrine treatment comprises the administration of an anti estrogen drug selected from the group comprising anastrozole, letrozole, exemestane, fulvestrant, toremifene and megasterol acetate.

22. A method for correlating the clinical outcome of a patient suffering from or at risk of developing a neoplastic disease with the presence or non-presence of a defect in expression levels of the RNA transcripts of at least one gene encoding for a hormone receptor selected from the group comprising estrogen receptor, progesterone receptor and/or androgen receptor, and/or the DNA amplification level of at least one gene encoding for a hormone receptor selected from the group comprising estrogen receptor, progesterone receptor and/or androgen receptor, and/or the presence or absence of at least one single nucleotide polymorphism of a hormone receptor selected from the group comprising estrogen receptor, progesterone receptor and/or androgen receptor, said method comprising the steps of:
a) obtaining a biological sample from said patient;
b) determining the status of at least one hormone receptor selected from the group comprising estrogen receptor, progesterone receptor and/or androgen receptor in said sample, using least two determinations selected from the group comprising:
aa) determining the expression level of the RNA transcripts of at least one gene encoding for a hormone receptor selected from the group comprising estrogen receptor, progesterone receptor and/or androgen receptor, and/or
bb) determining the DNA amplification level of at least one gene encoding for a hormone receptor selected from the group comprising estrogen receptor, progesterone receptor and/or androgen receptor, and/or
cc) determining the presence or absence of at least one single nucleotide polymorphism of a hormone receptor selected from the group comprising estrogen receptor, progesterone receptor and/or androgen receptor, and
c) correlating the pattern of expression level(s) of aa), amplification level(s) of bb) and/or the single nucleotide polymorphism(s) of cc) determined in step b) with said patient's data, said data being selected from the group consisting of etiopathology data, clinical symptoms, anamnesis data and/or data concerning the therapeutic regimen.

23. A method of selecting a therapy modality for a patient afflicted with a neoplastic disease, said method comprising the steps of:
a) obtaining a biological sample from said patient;
b) predicting from said sample, by the method according to any one of the aforementioned claims, therapeutic outcome for a plurality of individual modes of treatment; and
c) selecting a mode of treatment which is predicted to be successful in step b).

24. A method for adapting therapeutic regimen based on individualized risk assessment for a patient suffering from or at risk of developing a neoplastic disease, comprising the steps of:
a) obtaining a biological sample from said patient;
b) determining the status of at least one hormone receptor selected from the group comprising estrogen receptor, progesterone receptor and/or androgen receptor in said sample, using at least two determinations selected from the group comprising:
aa) determining the expression level of the RNA transcripts of at least one gene encoding for a hormone receptor selected from the group comprising estrogen receptor, progesterone receptor and/or androgen receptor, and/or
bb) determining the DNA amplification level of at least one gene encoding for a hormone receptor selected from the group comprising estrogen receptor, progesterone receptor and/or androgen receptor, and/or
cc) determining the presence or absence of at least one single nucleotide polymorphism of at least one gene encoding for a hormone receptor selected from the group comprising estrogen receptor, progesterone receptor and/or androgen receptor;
c) comparing the pattern of expression level(s) of aa), amplification level(s) of bb) and/or the single nucleotide polymorphism(s) of cc) determined in step b) with one or several reference pattern(s) of expression and/or amplification levels and/or single nucleotide polymorphisms; and
d) implementing therapeutic regimen targeting a hormone receptor selected from the group comprising estrogen receptor, progesterone receptor and/or androgen receptor in said patient from the outcome of the comparison in step c).

25. A kit useful for carrying out a method of any one of the aforementioned claims, comprising at least a pair of gene specific primers and/or probes each having a sequence sufficiently complementary to at least one gene or gene fragments or genomic nucleic acid sequence encoding for a at least one marker selected from the group comprising a hormone receptor selected from the group comprising estrogen receptor, progesterone receptor and/or androgen receptor for quantifying the expression of said at least one gene or gene fragment or genomic nucleic acid sequence, and/or their fractions, variants, homologues, derivatives, fragments, complements, hybridizing counterparts, or molecules sharing a sequence identity of at least 70%, preferably 95 %.

26. The kit according to claim 25 comprising specific primers and/or probes each having a sequence sufficiently complementary to at least one of the single nucleotide polymorphisms of estrogen receptor selected from the group of dbSNP Submission_ID comprising rs2071454, rs28462265, rs3138774, rs9371556, rs9340770, rs9340771, rs867239, rs867240, rs34418993, rs41291045, rs9340772, rs872921, rs2077647, rs9340773, rs746432, rs17847065, rs17847076, rs9340802, rs4986934, rs1801132, rs17847067, rs9341068, rs9341069, rs34840398, rs2228480, rs9340799, and/or rs2234693 and/or the single nucleotide polymorphisms of progesterone receptor selected from the group group dbSNP Submission_ID comprising rs34720665, rs11571142, rs11571141, rs10895068, rs2008112, rs518162, rs11571140, rs11571139, rs11224606, rs11571138, rs625942, rs11571137, rs35974050, rs11571136, rs500760, rs11571252, rs2020880, rs2020878, rs1042839, rs1042838, rs11571222, rs2020874, rs2020876, rs11571179, rs11571152, rs11571151, rs11571150, rs1379130, rs11571149, rs11571148, rs11571147, rs3740753, rs35747049, rs34852313, rs11571146, rs10160588, rs10160726, rs36055552, rs11571145, rs11571144, rs3740754, and/or rs11571143, and/or their fractions, variants, homologues, derivatives, fragments, complements, hybridizing counterparts, or molecules sharing a sequence identity of at least 70%, preferably 95 %.
